# FASCICULE DE BREVET EUROPEEN

(11) **EP 3 787 752 B1**
(45) Date de publication et mention de la délivrance du brevet: **06.03.2024**
(21) Numéro de dépôt: 19724557.4
(22) Date de dépôt: 19.04.2019
(51) Int. Cl.: A61Q 5/00, A61Q 5/12, A61K 8/92

(54) **COMPOSITION CAPILLAIRE COMPRENANT DES CIRES VEGETALES MODIFIEES**
HAARPFLEGEZUSAMMENSETZUNG MIT MODIFIZIERTEN PFLANZENWACHSEN
HAIRCARE COMPOSITION COMPRISING MODIFIED VEGETABLE WAXES

(30) Priorité: 02.05.2018 FR 1853765
(43) Date de publication de la demande: 10.03.2021
(73) Titulaire: GATTEFOSSE SAS, 69800 Saint Priest (FR)
(72) Inventeur: HUBICHE, Vincent, 69720 ST BONNET DE MURE (FR); RODIER, Jean-David, 69100 VILLEURBANNE (FR); LENNON, Paula, 69006 LYON (FR)
(74) Mandataire: Cabinet Laurent & Charras
(86) Numéro de dépôt international: PCT/FR2019/050935
(87) Numéro de publication internationale: WO 2019/211544

(56) Documents cités:
- FR-A1- 2 760 970
- FR-B1- 2 760 970
- US-A1- 2015 034 116
- Anonymous: "Bulletin 22 HYDRACIRE S - Jojoba Wax, Sunflower Wax & Mimosa Wax", INTERNET ARTICLE, 15 septembre 2009 (2009-09-15), XP055209746, Extrait de l'Internet: URL:http://alliance2u.com/pdf/Bulletin22.p df [extrait le 2015-08-26]
- Gattefossé: "3 Waxes, 3 Benefits, 1 Ingredient acticire ?", , 16 avril 2013 (2013-04-16), XP055279702, Extrait de l'Internet: URL:http://www.scsformulate.co.uk/wp-conte nt/uploads/2015/08/Acticire-Brochure.pdf [extrait le 2016-06-10]
- DATABASE GNPD [Online] MINTEL; 7 mars 2014 (2014-03-07), anonymous: "Moisturizing Hair Mask", XP055539088, extrait de www.gnpd.com Database accession no. 2338412

## Description

### DOMAINE DE L'INVENTION

L'invention concerne l'utilisation d'une composition capillaire comprenant un mélange de cires modifiées pour le contrôle du volume des cheveux, le contrôle des frisottis, la facilité de peignage, l'amélioration de la résistance à l'électricité statique et de l'hydrophobicité des cheveux, lorsqu'il est utilisé dans des formules de soin du cheveu, à rincer ou non.

### ETAT DE LA TECHNIQUE

Les cheveux sont constamment exposés à des conditions extrinsèques difficiles telles que le rayonnement ultra-violet, la pollution, l'humidité, des dommages chimiques (nettoyant, blanchissant, permanente), la chaleur (sèche-cheveux, lisseur, friseur, ...), ou encore des dommages mécaniques (peignage, ...).

En outre, la quantité de cheveux, tout comme leur qualité, peut décroitre au cours du temps en raison de l'âge et/ou d'autres facteurs, tels que le renouvellement cellulaire déficient au niveau du scalp ou encore une production excessive ou insuffisante de sébum.

Ces différents facteurs peuvent conduire à une nature de cheveux plus fins et/ou plus fragiles et/ou nuire à l'aspect visuel et/ou au toucher des cheveux.

Par exemple, en conditions très humides, les cheveux ont tendance à absorber de l'eau, ce qui conduit à une perte de leur forme, les rend indisciplinés et entraine l'apparition de frisottis. A l'inverse, en conditions très sèches, les cheveux vont se dessécher, devenir plus fragiles et d'aspect moins brillant. Il est donc important de renforcer l'hydrophobie des cheveux afin de limiter l'influence des conditions environnementales.

Afin de limiter ou prévenir les dommages causés aux cheveux, les consommateurs recherchent des produits de soins capillaires, souvent appelés conditionneurs, qui vont permettre de protéger les cheveux des agressions extérieures et d'améliorer leur apparence. Ces produits de soin capillaire sont principalement constitués de silicone, d'huiles végétales et de conditionneurs quaternaires.

Les silicones permettent d'améliorer le peignage, procurent de la douceur et un aspect brillant. Toutefois, ils présentent plusieurs inconvénients. Ce sont des composés synthétiques qui sont peu biodégradables. En outre, il s'agit de composés hydrophobes et lipophobes. En conséquence, ils sont plus difficiles à enlever lors du nettoyage par un shampoing. Par ailleurs, au fur et à mesure des utilisations, une accumulation des silicones se fait sur les cheveux, les rendant plus lourds, ce qui entrave, notamment, le coiffage.

Les huiles et beurres végétaux peuvent remplacer en partie les silicones. Ils ont pour effets de nourrir le cheveu, l'hydrater et l'assouplir. Mais, ces composés conduisent à graisser les cheveux et affichent une moindre performance. D'autre part, les huiles et beurres végétaux sont des triglycérides insaturés qui s'oxydent facilement, ce qui pose un problème au niveau de la stabilité des formules. De plus, en formulation de type émulsion, ces huiles ne sont pas faciles à stabiliser. C'est pourquoi, on les trouve surtout sous forme d'huile liquide capillaire.

Enfin, les conditionneurs quaternaires ou polymères cationiques, sont destinés à faciliter le peignage et lutter contre l'électricité statique par la charge positive qu'ils apportent, neutralisant ainsi les charges négatives des cheveux. Néanmoins, ces composés peuvent être irritants pour la peau, sont partiellement non biodégradables et sont pour certains, des allergènes.

Le document US2015034116A1 décrit une composition à base d'un mélange de cires telles que les cires d'abeilles et d'ozokérite.

A la connaissance du Demandeur, aucune composition capillaire n'a été mise au point qui ne présente pas les inconvénients mentionnés précédemment.

### DESCRIPTION DETAILLEE DE L'INVENTION

Le Demandeur a constaté que, de manière tout à fait surprenante, le produit de la réaction issue d'un mélange comprenant la cire de jojoba, la cire de tournesol et le polyglycérol-3 apporterait des bénéfices sur le contrôle du volume des cheveux, le contrôle des frisottis, la facilité de peignage, l'amélioration de la résistance à l'électricité statique et de l'hydrophobicité des cheveux, lorsqu'il est utilisé dans des formules de soin du cheveu, à rincer ou non.

Par frisottis, on désigne des cheveux qui ne restent pas en forme dans la coiffure, en d'autres termes, il s'agit de cheveux « indisciplinés ».

Le document EP 1 933 806 A1 du Demandeur, décrit un procédé de préparation d'un excipient par la transformation simultanée entre elles d'une cire solide et d'une cire liquide avec un polyol. Le mélange de cire de jojoba, cire de tournesol et polyglycérol-3 n'est pas décrit.

Le Demandeur a constaté qu'il est possible d'obtenir une composition cosmétique, avantageusement une composition capillaire, comprenant un dérivé de cires obtenu par la réaction de transestérification simultanée entre la cire de jojoba et la cire de tournesol, en présence du polyglycérol-3.

Cette composition capillaire possède des effets équivalents, voire supérieurs, sur les cheveux, sans les inconvénients liés à la nature des composés de l'art antérieur (non biodégradable, difficulté de nettoyage, lourdeur des cheveux, instabilité ou encore cristallisation en formulation).

Ainsi, la présente invention a pour objet une composition capillaire comprenant un dérivé de cires obtenu par la réaction de transestérification simultanée d'un mélange comprenant de la cire de jojoba et de la cire de tournesol en présence du polyglycérol-3 pour les utilisations telles que définies dans les revendications.

La cire de jojoba est un liquide à l'aspect huileux, visqueux, légèrement jaunâtre, peu odorant, obtenu par pression des graines de *Simmondsia chinensis.* La cire de jojoba est composée essentiellement de monoesters aliphatiques insaturés contenant 38 à 44 carbones. Ces esters sont constitués d'acides gras linéaires insaturés et d'alcool gras linéaires insaturés.

La cire de tournesol constitue une fine pellicule autour des graines, c'est-à-dire la coque de la graine de tournesol ou *Helianthus annuus.* Elle est extraite de l'huile de tournesol par filtration après une étape de précipitation ou « winterization ». Il ne s'agit donc pas d'huile de tournesol, généralement obtenue par extraction par pression ou par solvant à partir des graines de tournesol.

En particulier, la cire de tournesol n'a pas la même composition que l'huile de tournesol qui est constituée de triglycérides. La cire de tournesol est composée de monoesters aliphatiques saturés contenant 42 à 62 carbones. Ces esters sont constitués d'acides gras linéaires saturés et d'alcools gras linéaires saturés.

Alternativement à la forme non hydrolysée, la cire de tournesol peut, en outre, se présenter sous une forme hydrolysée en totalité ou partiellement. Il s'agit dans ce cas-là de mettre en oeuvre des acides gras linéaires saturés et des alcools gras linéaires saturés. Ceux-ci vont se recombiner par réaction d'estérification lors de la mise en oeuvre de cette cire avec la cire de jojoba en présence de polyglycérol-3.

Par « cire de tournesol partiellement hydrolysée » on désigne une hydrolyse de la cire de tournesol entre 50% et 100%, avantageusement entre 75% et 100%.

Le mélange de cire de jojoba et de cire de tournesol, en présence de polyglycérol-3 peut, en outre, éventuellement comprendre au moins un alcool gras saturé libre ou estérifié, avantageusement contenant au moins 22 atomes de carbones et/ou un acide gras saturé, libre ou estérifié, avantageusement contenant au moins 22 atomes de carbones.

Le polyglycérol-3 est un polyol de formule C₉H₂₀O₇, dont la masse molaire est d'environ 240 g/mol. Ce composé porte, en moyenne, 5 fonctions alcool (OH).

Le Demandeur a constaté que le produit de réaction simultanée entre la cire de jojoba, la cire de tournesol, éventuellement partiellement ou totalement hydrolysée, en présence de polyglycérol-3, présente une dureté plus faible que le produit issu du mélange des deux cires.

Ce comportement différent pourrait provenir, selon le Demandeur, de la présence de nouveaux dérivés de polyol n'existant pas lorsque les produits sont mélangés séparément ainsi que de nouveaux esters aliphatiques. Le dérivé de polyol correspond à une structure dans laquelle toute ou partie des fonctions OH du polyglycérol-3 sont estérifiées avec les acides gras linéaires saturés de la cire de tournesol et les acides gras linéaires insaturés de la cire de jojoba. Des alcools gras linéaires insaturés de la cire de jojoba et les alcools gras linéaires saturés de la cire de tournesol sont alors libérés lors de cette réaction. Les nouveaux esters aliphatiques sont constitués des acides gras linéaires insaturés de la cire du jojoba combinés avec les alcools gras linéaires saturés de la cire de tournesol et des acides gras linéaires saturés de la cire de tournesol combinés avec les alcools gras linéaires insaturés de la cire de jojoba.

Par cette réaction de transestérification, une redistribution aléatoire des acides et alcools composant les cires est obtenue. Le dérivé de cires contient donc, en fin de réaction, des esters d'acides linéaires mono-insaturés ou saturés et d'alcools linéaires saturés, des esters d'acides linéaires saturés et d'alcools linéaires mono-insaturés ou saturés, des alcools libres linéaires saturés, des alcools libres linéaires mono-insaturés saturés et les esters de polyol précédemment décrits, en plus des constituants de départ qui n'auraient pas réagi.

Avantageusement, le rapport massique cire de jojoba/cire de tournesol représente entre 50/50 et 90/10, avantageusement entre 60/40 et 80/20, de préférence 70/30.

De préférence, le rapport massique cire de jojoba et cire de tournesol/polyglycérol-3 représente entre 99/1 et 80/20, avantageusement entre 98/2 et 90/10, de préférence 95/5.

Selon un mode de réalisation de l'invention, le dérivé de cires, obtenu par la réaction de transestérification simultanée d'un mélange comprenant de la cire de jojoba, de la cire de tournesol en présence du polyglycérol-3, représente entre 0,01 et 20% en poids de la composition, avantageusement entre 0,1 et 5%.

Le dérivé de cires obtenu selon la réaction de transestérification mentionnée ci-dessus, possède un comportement d'émulsionnant eau dans huile ainsi qu'une capacité à absorber l'eau.

Selon la présente invention, le dérivé de cires est obtenu par la réaction de transestérification simultanée d'un mélange comprenant de la cire de jojoba et de la cire de tournesol en présence du polyglycérol-3 et d'au moins un catalyseur.

Le au moins un catalyseur est choisi parmi le groupe comprenant les hydroxydes, les alkoxydes alcalins et les alcalino terreux.

Avantageusement, le au moins un catalyseur est choisi parmi le groupe comprenant la soude, la potasse (en solution alcoolique, aqueuse ou sous forme solide), l'hydroxyde de calcium, le carbonate de potassium ou de sodium.

En pratique, la réaction est conduite à une température comprise entre 150°C et 240°C, avantageusement entre 180 et 220°C.

L'invention a également pour objet la composition capillaire, telle que définie précédemment, pour utilisation pour :
- contrôler le volume capillaire ;
- un effet anti-frisottis ;
- le démêlage capillaire, avantageusement pour faciliter le peignage ;
- améliorer la définition et le maintien des boucles capillaires ;
- lutter contre l'électricité statique générée au niveau capillaire ; et
- augmenter l'hydrophobie capillaire.

La composition capillaire comprenant le dérivé de cires selon l'invention peut être formulée sous toutes les formes galéniques normalement utilisées pour une application capillaire, notamment par exemple sous forme anhydre, sous forme d'une émulsion huile-dans-eau, d'une émulsion eau-dans-huile, d'une émulsion multiple, d'une microémulsion, d'une nanoémulsion, d'un gel ou d'une solution hydro-alcoolique.

Cette composition peut être plus ou moins fluide et se présenter sous forme d'une crème, d'une pommade, d'un lait, d'une lotion, d'une solution, d'un sérum, d'un conditionneur, d'un soin, d'une cire, d'un masque ou d'un gel.

En outre, la composition capillaire comprenant le dérivé de cires selon l'invention est pour application sur les cheveux et d'autres types de poils, en l'espèce, la barbe, la moustache, les cils ou encore les sourcils.

La composition capillaire peut également comprendre les excipients habituellement utilisés dans les domaines capillaires, tels que les matières grasses, les tensioactifs détergents et/ou conditionnants, les émulsionnants et co-émulsionnants, les gélifiants hydrophiles ou lipophiles, les conservateurs, les antioxydants, les solvants, les agents exfoliants, les parfums, les charges, les filtres solaires hydrophiles et lipophiles, les matières colorantes, les neutralisants, les agents pro-pénétrants, et les polymères. Ces types d'excipients sont tous bien connus de l'Homme du métier.

En pratique, les quantités de ces différents excipients sont celles classiquement utilisées dans les domaines considérés, et la somme des excipients représente de préférence 0,01% à 99,9% du poids total de la composition.

Comme matières grasses appropriées, on peut citer les huiles minérales, les huiles d'origine animale (telle la lanoline), les huiles et beurres végétales, les huiles de synthèse (comme par exemple les polydécènes hydrogénés), les éthers (comme par exemple l'éther dicaprylique), les esters (comme par exemple le myristate d'octyldodécyle, le palmitate d'isopropyle, le caprylo-caprate de triglycéryle) et les huiles fluorées. On peut utiliser comme matières grasses des alcools gras, des acides gras et des gommes.

Comme tensioactifs détergents et/ou conditionnants appropriés, on peut citer les tensioactifs non ioniques, anioniques, cationiques ou amphotères, et leurs mélanges, tels que par exemple les alkylsulfates, les alkyléthersulfates tels que le lauryl éther sulfate de sodium, les alkyl bétaïnes telles que la cocamidopropylbétaïne, ou les sels d'ammonium quaternaires.

Comme émulsionnants et co-émulsionnants appropriés, on peut citer par exemple les esters de sucrose et d'acide gras, les esters de sorbitane et d'acide gras, les esters d'acide gras et de sorbitane oxyéthylénés, les éthers d'alcool gras et de polyéthylène glycol, les esters de glycérol et d'acide gras, les alkyl sulfates, les alkyl éther sulfates, les alkyl phosphates, les alkyl polyglucosides, les diméthicone copolyols.

Comme gélifiants hydrophiles appropriés, on peut citer par exemple les polymères carboxyvinyliques (carbomers), les copolymères acryliques tels que les copolymères d'acrylates/alkylacrylates, les polyacrylamides, les polysaccharides tels que la gomme xanthane, la gomme guar, les gommes naturelles telles que la gomme de cellulose et dérivés, les amidons et leurs dérivés, les argiles et les copolymères d'acide 2-acrylamido-2-méthylpropane.

Comme gélifiants lipophiles appropriés, on peut citer par exemple les argiles modifiées comme les bentones, les sels métalliques d'acides gras, la silice hydrophobe et l'éthylcellulose.

Comme conservateurs appropriés, on peut citer par exemple les acides benzoïque, sorbique, propionique, salicylique, déhydroacétique et leurs sels, l'alcool benzylique, l'éthylhexylglycérine, les parabènes, leurs sels et esters, le triclosan, l'imidazolidinyl urée, le 5-phénoxyéthanol, la diméthylol diméthyl (DMDM) hydantoïne, le diazolidinyl urée, la chlorphénésine.

Comme antioxydants appropriés, on peut citer par exemple les agents chélatants tels que l'éthylène diamine tétra-acétique (EDTA) et ses sels, le métabisulfite de sodium, les acides salicylique, ascorbique et citrique et leurs sels, le tartrate de sodium, le gluconate de sodium, les caroténoïdes et les tocophérols.

Comme solvants utilisables dans la composition capillaire (distinct du solvant d'extraction), on peut citer l'eau, avantageusement l'eau déminéralisée, l'éthanol, la glycérine, le propylène glycol, le propanediol, le butylène glycol, le sorbitol.

Comme agents exfoliants appropriés, on peut citer par exemple les exfoliants chimiques tels que les acides alpha-hydroxylés (AHA), et les exfoliants physiques tels que les poudres naturelles ou synthétiques.

Comme charges appropriées, on peut citer par exemple le talc, le kaolin, le mica, la séricite, le magnésium carbonate, l'aluminium silicate, le magnésium silicate, les poudres organiques telles que le nylon.

Comme colorants appropriés, on peut citer par exemple les colorants lipophiles, les colorants hydrophiles, les pigments et les nacres habituellement utilisés dans les compositions capillaires, et leurs mélanges.

Comme neutralisants appropriés, on peut citer par exemple la soude, la triéthanolamine, l'aminométhyl propanol, l'hydroxyde de potassium.

Comme agents pro-pénétrants appropriés, on peut citer par exemple les alcools et glycols (éthanol, propylène glycol), l'éthoxydiglycol, les alcools et acides gras (acide oléique), les esters d'acides gras, le diméthyl isosorbide.

Outre le composé fonctionnel selon l'invention, en l'espèce le dérivé de cires, la composition de l'invention peut également contenir des actifs. Comme actifs appropriés, on peut citer par exemple les anti-pelliculaires, les décolorants, les agents restructurants, les protecteurs thermiques, les anti-radicalaires ou plus généralement les antioxydants, les émollients, les hydratants, les anti-séborrhéiques, les anti-inflammatoires, les agents kératolytiques et/ou desquamants, les agents drainants, les agents anti-irritants, les agents apaisants, les vitamines et leurs mélanges, les agents matifiants et de brillance, les cicatrisants, les antiseptiques, les huiles essentielles et les protéines telle que la kératine. La manière dont l'invention peut être réalisée et les avantages qui en découlent ressortiront mieux des exemples de réalisation qui suivent, donnés à titre indicatif et non limitatif, à l'appui des figures annexées.

### FIGURES

La figure 1 représente la variation du volume des cheveux, en pourcentage, selon différentes formulations.
La figure 2 représente la variation de la quantité de frisottis en pourcentage, selon différentes formulations.
La figure 3 représente la variation de la force de peignage des cheveux, en pourcentage, selon différentes formulations.
La figure 4 représente la variation de la taille des mèches de cheveux bouclées, en pourcentage, selon différentes formulations.
La figure 5 représente la variation de la résistance à l'électricité statique selon différentes formulations.
La figure 6 représente une comparaison, des formes liquides à 80°C, d'un dérivé de cires selon l'invention (bécher de gauche) avec un dérivé de cires de l'art antérieur (bécher de droite).

### EXEMPLES DE REALISATION DE L'INVENTION

### Exemple 1 : Obtention d'un dérivé de cires

Le dérivé de cires est obtenu par transestérification simultanée de la cire de jojoba et de la cire de tournesol non hydrolysée, en présence du polyglycérol-3.

Composition du mélange réactionnel de départ :

| | |
|---|---|
| Cire de jojoba | 300 g |
| Cire de tournesol (non hydrolysée) | 177 g |
| Polyglycérol-3 | 22 g |
| Hydroxyde de sodium | 1 g |

Les produits sont introduits dans un réacteur agité d'un litre. La réaction se déroule à 210°C, pendant 12 heures sous un inertage à l'azote. A la fin du palier de température, le catalyseur est neutralisé. Le dérivé de cires est récupéré, après avoir séparé une partie du polyglycérol n'ayant pas réagi, puis filtré pour éliminer les sels de neutralisation. La quantité récupérée de dérivé de cires est d'environ 475 g. Le produit obtenu est de couleur beige et d'une consistance molle. L'indice d'hydroxyle est d'environ 33 mg KOH/g et le point de goutte de 62°C.

### Exemple 2 : Obtention d'un dérivé de cires

Le dérivé de cires est obtenu par transestérification simultanée de la cire de jojoba et de la cire de tournesol partiellement hydrolysée, en présence du polyglycérol-3.

Composition du mélange réactionnel de départ :

| | |
|---|---|
| Cire de jojoba | 301 g |
| Cire de tournesol hydrolysée à 82% | 174 g |
| Polyglycérol-3 | 22 g |
| Hydroxyde de sodium | 0,5 g |

Les produits sont introduits dans un réacteur agité d'un litre. La réaction se déroule à 215°C, pendant 6 heures sous un inertage à l'azote. L'indice d'acide du mélange réactionnel est de 24 mg KOH/g. A la fin du palier de température, l'indice d'acide est de 0,5 mg KOH/g. Le catalyseur est neutralisé. Le dérivé de cires est récupéré, après avoir séparé une partie du polyglycérol n'ayant pas réagi, puis filtré pour éliminer les sels de neutralisation. La quantité récupérée de dérivé de cires est d'environ 488 g. Le produit obtenu est de couleur marron et d'une consistance plus dure que celle de l'exemple 1. Le point de goutte mesuré est de 66,1°C.

### Exemple 3 : Obtention d'un dérivé de cires

Le dérivé de cires est obtenu par transestérification simultanée de la cire de jojoba et de la cire de tournesol non hydrolysée, en présence du polyglycérol-3, d'acides et d'alcools gras linéaires saturés à longues chaines.

Composition du mélange réactionnel de départ :

| | |
|---|---|
| Cire de jojoba | 305 g |
| Cire de tournesol (non hydrolysée) | 135 g |
| Acide béhénique (C22) | 21g |
| Alcool béhénylique (C22) | 21g |
| Polyglycérol-3 | 17 g |
| Hydroxyde de sodium | 1 g |

Les produits sont introduits dans un réacteur agité d'un litre. La réaction se déroule à 215°C, pendant 12 heures sous un inertage à l'azote. A la fin du palier de température, l'indice d'acide est de 0,1 mg KOH/g. Le catalyseur est neutralisé. Le dérivé de cires est récupéré puis filtré pour éliminer les sels de neutralisation. La quantité récupérée de dérivé de cires est d'environ 490 g. Le produit obtenu est de couleur jaune clair et d'une consistance molle. Le point de goutte mesuré est de 62,1°C et l'indice d'hydroxyle de 42 mg KOH/g.

### Exemple 4 : Formulations testées

| | | **Formule A** | **Formule B** | **Formule C** | **Formule D** |
|---|---|---|---|---|---|
| **Nom de l'ingrédient** | **Nom INCI** | **% ingrédient** | | | |
| Eau déminéralisée | Water | 88,3 | 86,3 | 86,3 | 86,3 |
| Emulium^{®} Delta | Cetyl alcohol, Glyceryl stéarate, PEG-75 stéarate, Ceteth-20, Steareth-20 | 6,0 | 6,0 | 6,0 | 6,0 |
| Dérivé de cires de l'exemple 3 | | - | 2,0 | - | |
| Huiles de silicone | Dimethicone, Dimethiconol | - | - | 2,0 | |
| Ammonium quaternaires | Behentrimonium chloride | - | - | - | 2,0 |
| Huile minérale AAB2 | Mineral oil | 3,0 | 3,0 | 3,0 | 3,0 |
| Schercemol^{™} dis ester | Diisopropyl sebacate | 2,0 | 2,0 | 2,0 | 2,0 |
| PE 9010 | Phenoxyethanol, Ethylhexylglycerin | 0,7 | 0,7 | 0,7 | 0,7 |
| **TOTAL** | | **100** | **100** | **100** | 100 |

La formule A correspond à une formule placebo, la formule B contient le dérivé de cires selon l'invention, la formule C contient des silicones et la formule D contient des quaternaires.

### Exemple 5 : Mesure du volume des cheveux

A l'aide de l'appareil Rumba de Bossa Nova Technologie, dédié à la mesure *in vitro* de l'orientation des fibres capillaires, la variation du volume d'une mèche de cheveux décolorée (cheveux brun bouclés) et traitée par une formule (0,5g de formule pour 1g de cheveux) lorsqu'elle a été stockée 4h en conditions humides (30°C / >75% HR) a été mesurée.

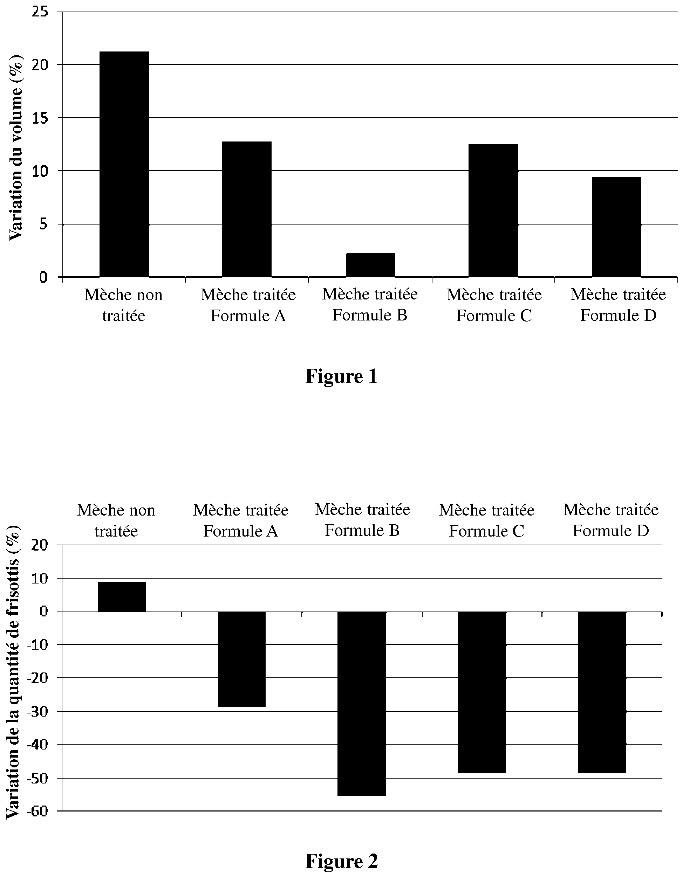

Les résultats sont représentés par la figure 1.

**Tableau 1 : Mesure de la variation du volume d'une mèche de cheveux**

| | Variation du volume (%) |
|---|---|
| Mèche non traitée | +21,2 |
| Mèche traitée avec Formule A (placebo) | +12,7 |
| Mèche traitée avec Formule B (dérivé de cires de l'exemple 3) | +2,2 |
| Mèche traitée avec Formule C (silicones) | +12.5 |
| Mèche traitée avec Formule D (quaternaires) | +9,4 |

En conditions humides, une mèche de cheveux non traitée a tendance à gonfler, en raison de la formation des ponts hydrogènes entre les fibres dus à la présence d'eau (+21,2%). L'application de la formule B, contenant 2% du dérivé de cires de l'exemple 3, permet de limiter drastiquement ce gonflement (+2,2%), beaucoup plus que la formule A, placebo, (+12,7%), celle contenant les silicones, formule C, (+12,5%) et celle contenant les quaternaires, formule D (+9,4%).

### Exemple 6 : Mesure de l'effet anti-frisottis

A l'aide de l'appareil Rumba de Bossa Nova Technologie, l'apparition de frisottis a été mesuré après lavage avec un shampoing standard et application sur une mèche de cheveux décolorée (cheveux brun bouclés) d'une formule (0,5g de formule pour 1g de cheveux) puis après stockage 1h dans des conditions standardisées (23°C / 50% d'humidité relative ; HR).

Les résultats sont représentés par la figure 2.

**Tableau 2 : Mesure de la variation de la fréquence des frisottis**

| | Variation des frisottis (%) |
|---|---|
| Mèche non traitée | +8,8 |
| Mèche traitée avec Formule A (placebo) | -28,7 |
| Mèche traitée avec Formule B (dérivé de cires de l'exemple 3) | -55,3 |
| Mèche traitée avec Formule C (silicones) | -48.3 |
| Mèche traitée avec Formule D (quaternaires) | -48.5 |

En conditions standardisées (atmosphère légèrement sèche), une mèche de cheveux lavée, mais non traitée ensuite, aura tendance à continuer de former des frisottis (+8,8%). L'application de la formule B, contenant 2% de dérivé de cires de l'exemple 3, permet de diminuer la quantité de frisottis formés suite au shampoing (-55,3%), de manière beaucoup plus efficace que la formule A, placebo, (-28,7%), et avec des performances équivalentes à la formule C, contenant les silicones, (-48,3%) et à la formule D, contenant les quaternaires (-48,5%).

### Exemple 7 : Mesure de la peignabilité

A l'aide d'un texturomètre TA.XTPlus de Texture Technologies équipé d'un système de peignage, la force nécessaire au peignage d'une mèche de cheveux décolorée (brun bouclés) avant et après traitement de la mèche par une formule (0,5g de formule pour 1g de cheveux) a été mesurée. Après traitement chaque mèche est stockée 30 minutes à 23°C / 50% HR avant la mesure, afin de les laisser sécher.

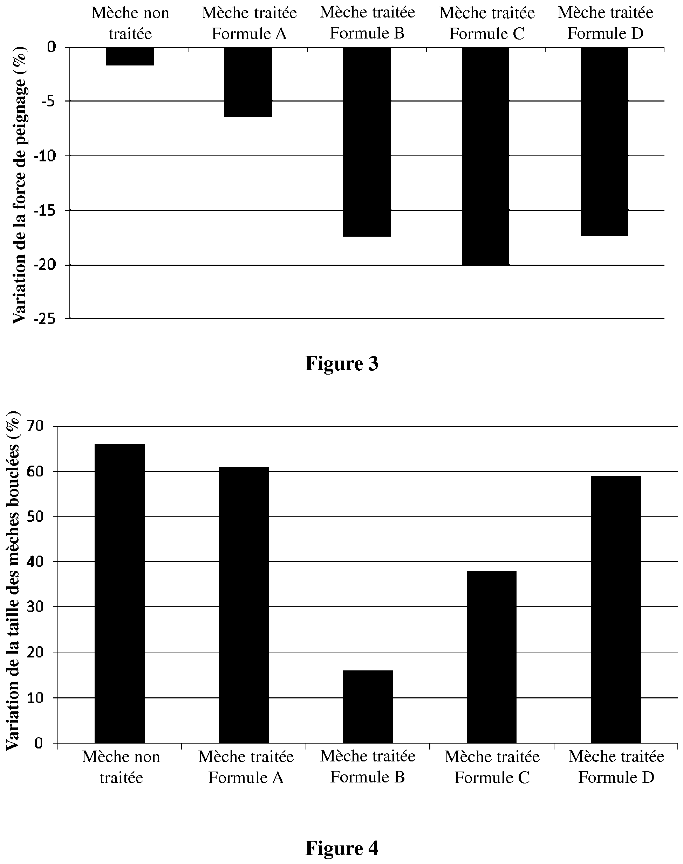

Les résultats sont représentés par la figure 3.

**Tableau 3 : Mesure de la variation de la force de peignage**

| | Variation de la force de peignage (%) |
|---|---|
| Mèche non traitée | -1,7 |
| Mèche traitée avec Formule A (placebo) | -6,5 |
| Mèche traitée avec Formule B (dérivé de cires de l'exemple 3) | -17,5 |
| Mèche traitée avec Formule C (silicones) | -20,1 |
| Mèche traitée avec Formule D (quaternaires) | -17.3 |

La mèche traitée avec la formule B, contenant le dérivé de cires de l'exemple 3, montre une meilleure facilité de peignage (-17,5%), c'est-à-dire qu'une force moins importante est nécessaire pour peigner la mèche. Au contraire, la formule B, placebo, montre une plus faible réduction (-6,5%). La performance obtenue avec la formule selon l'invention est équivalente à celle obtenue avec la formule D, contenant les quaternaires, (-17,3%) et proche de celle de la formule C, contenant les silicones, (-20,1%).

### Exemple 8 : Mesure du maintien des boucles

Des mèches de cheveux (brun standard) ont été décolorées plus lavées, avant d'être traitées par une formule (0,5g de formule pour 1g de cheveux) pendant 3 minutes puis rincées à l'eau. Les mèches sont ensuite placées sur des bigoudis puis laissées sécher pendant 24h en conditions sèches (23°C / 30% HR). Après séchage, les bigoudis sont retirés, la taille de la mèche est mesurée avant puis après stockage 4h en conditions humides (34°C / 55% HR).

Les résultats sont représentés par la figure 4.

**Tableau 4 : Mesure de la variation de la taille des mèches bouclées**

| | Variation de la taille des mèches bouclées |
|---|---|
| Mèche non traitée | +66 % |
| Mèche traitée avec Formule A (placebo) | +61 % |
| Mèche traitée avec Formule B (dérivé de cires de l'exemple 3) | +16 % |
| Mèche traitée avec Formule C (silicones) | +38% |
| Mèche traitée avec Formule D (quaternaires) | +59% |

Alors qu'une mèche non traitée bouclée va beaucoup s'allonger au bout de 4h (+66%), la mèche traitée par la formule B, contenant le dérivé de cires de l'exemple 3, est très peu déformée (+16%). La formule A, placebo, n'a quasiment pas d'effet (+61%), tout comme la formule D, contenant les quaternaires, (+59%). Enfin la formule C, contenant les silicones, montre une moindre performance (+38%).

### Exemple 9 : Mesure de la résistance à l'électricité statique

Des mèches de cheveux (brun standard) ont été lavées, avant d'être traitées par une formule (0,5g de formule pour 1g de cheveux) pendant 3 minutes puis rincées à l'eau. Les mèches sont ensuite placées pendant 24h en conditions sèches (23°C / 30% HR). Après séchage, un ballon de baudruche gonflé est passé en faisant deux allers-retours sur la mèche afin de générer de l'électricité statique (simulant un air très sec, phénomène courant en hiver). On évalue ensuite visuellement l'aspect de chaque mèche.

L'électricité statique générée sur les cheveux entraine une répulsion entre les fibres qui se trouvent chargées.

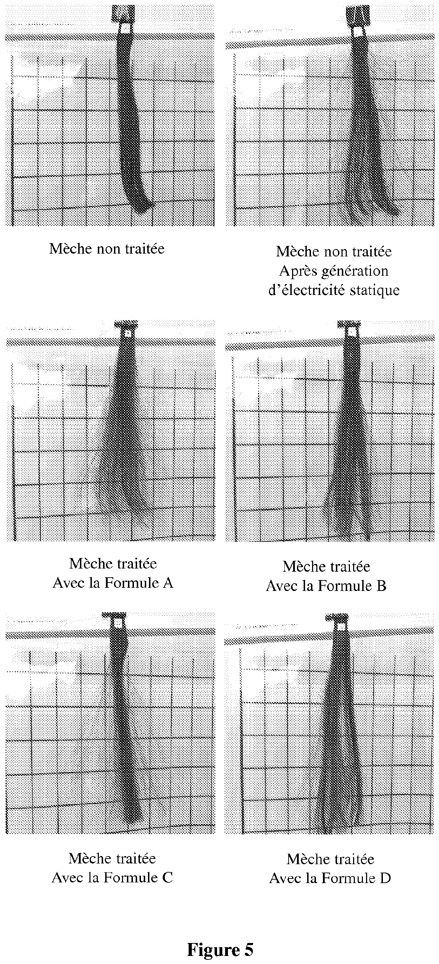

Les résultats sont représentés par la figure 5.

L'application de la formule B, contenant le dérivé de cires de l'exemple 3, permet de limiter la capacité des cheveux à se charger électriquement et ainsi de se repousser. La performance obtenue est équivalente à ce que l'on obtient avec la formule D, contenant les quaternaires. La formulation C, contenant les silicones, n'a pas d'effet sur l'électricité statique.

### Exemple 10 : Mesure de l'hydrophobicité

### i) Eau déminéralisée

Afin de quantifier l'hydrophobie apportée par le dérivé de cires de l'exemple 3, les différentes formules ont été appliquées sur une lame de verre à l'aide d'une réglette de 30µm (« haltère à laque ») puis laisser sécher 3h en conditions sèches (23°C / 30% HR). A l'aide d'un goniomètre ILMS de GBX instrument, une goutte d'eau déminéralisée a été déposée à la surface et l'angle de contact de la goutte d'eau a été mesuré sachant que plus il est grand plus le support est hydrophobe.

Par « eau déminéralisée», on désigne une eau exempte de minéraux et de particules polluantes.

La formule A, placebo, produit un angle de contact de 62° et la formule C, contenant les silicones, produit un angle de contact de 57°, équivalent au placebo. La formule D, contenant les quaternaires (très hydrophiles), produit un angle de 27°. Enfin, la formule B, contenant le dérivé de cires selon l'exemple 3, produit un angle de contact de 85°, montrant le gain d'hydrophobie apporté par l'utilisation du dérivé de cires de l'exemple 3.

### ii) Eau polluée

Afin de quantifier l'intérêt du dérivé de cires selon l'invention pour diminuer le contact et l'adhésion des polluants sur les cheveux, un test selon le protocole de l'exemple 10 i) a été utilisé et une goutte d'eau polluée a été déposée à la surface de la lame de verre.

Par « eau polluée », on désigne une eau contenant des particules de pollution collectées dans l'atmosphère urbaine (Urban dust NIST1649B ; Sigma-Aldrich).

Les résultats montrent une tendance similaire à ce qui est observé avec une goutte d'eau déminéralisée, à savoir, une plus grande hydrophobie apportée par l'utilisation du dérivé de cires de l'exemple 3 en comparaison avec les autres formules testées.

### Exemple 11 : Comparaison de l'aspect visuel d'un dérivé de cires selon l'invention par rapport à un dérivé de cires de l'art antérieur

La formulation du dérivé de cires selon l'invention est celle de l'exemple 3.

La formulation du dérivé de cires selon l'art antérieur a pour composition du mélange réactionnel de départ :

| | |
|---|---|
| Cire de jojoba | 571g |
| Cire de candellila | 123g |
| Cire de riz | 122g |
| Polyglycérol-3 | 144g |
| Carbonate de potassium | 40g |

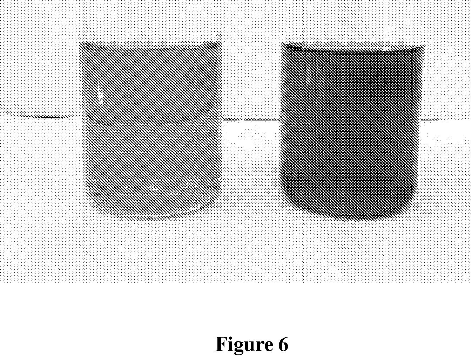

Les dérivés de cires sous forme liquide à 80°C sont représentés par la figure 6.

Le dérivé selon l'invention, obtenu à partir des cires de jojoba et de tournesol en présence de polyglycérol-3 est beaucoup moins coloré (figure 6, bécher de gauche) que le dérivé de l'art antérieur obtenu à partir des cires de jojoba, de riz et de candelilla en présence de polyglycérol-3 (figure 6, bécher de droite).

Selon le Demandeur, les cires de riz et de candellila se colorent pendant la synthèse et le dérivé qui en découle apporte alors une coloration aux formulations mettant en oeuvre ce dérivé de cires, impossible à masquer si le formulateur n'utilise pas d'autres ingrédients colorants tels que des pigments. Les qualités esthétiques du produit final sont donc impactées.

Cette altération esthétique du produit capillaire n'est pas observée avec l'utilisation en formulation du dérivé de cires selon l'invention.

### Exemple 12 : Exemples de formules

### Crème de nuit hydratante pour les cheveux

| **Nom INCI** | **% Matière** |
|---|---|
| WATER | 55,56 |
| GLYCERIN | 6,00 |
| INULIN | 1,50 |
| CYAMOPSIS TETRAGONOLOBA GUM | 1,30 |
| MICROCRYSTALLINE CELLULOSE, CELLULOSE GUM | 1,00 |
| WATER | 4,00 |
| SODIUM STEAROYL GLUTAMATE | 0,20 |
| POLYGLYCERYL-6 DISTEARATE, JOJOBA ESTERS, POLYGLYCERYL-3 BEESWAX, CETYL ALCOHOL | 2,50 |
| CRAMBE ABYSSINICA SEED OIL | 5,00 |
| **Dérivé de cires de l'exemple 3** | **2,50** |
| C10-18 TRIGLYCERIDES | 4,00 |
| OCTYLDODECYL MYRISTATE | 3,00 |
| MANGIFERA INDICA (MANGO) SEED BUTTER | 2,00 |
| HYDROGENATED ETHYLHEXYL OLIVATE, HYDROGENATED OLIVE OIL UNSAPONIFIABLES | 2,00 |
| TOCOPHEROL | 0,04 |
| WATER, MANGIFERA INDICA (MANGO) FRUIT EXTRACT | 3,00 |
| DMDM HYDANTOIN, IODOPROPYNYL BUTYLCARBAMATE | 0,40 |
| HYDROLYZED SOY PROTEIN | 5,00 |
| PARFUM | 1,00 |
| **TOTAL** | **100** |

### Masque capillaire fortifiant

| **Nom INCI** | **% Matière** |
|---|---|
| WATER | 64,6 |
| MICROCRYSTALLINE CELLULOSE, CELLULOSE GUM | 2,5 |
| GLYCERIN | 2,0 |
| XANTHAN GUM | 0,3 |
| INULIN | 2,5 |
| SODIUM STEAROYL GLUTAMATE | 0,3 |
| WATER | 10,0 |
| POLYGLYCERYL-6 DISTEARATE | 1,5 |
| GLYCERYL STEARATE | 1,5 |
| CETYL ALCOHOL | 1,0 |
| C10-18 TRIGLYCERIDES | 1,0 |
| THEOBROMA CACAO (COCOA) SEED BUTTER | 2,0 |
| **Dérivé de cires de l'exemple 3** | **2,0** |
| CANDELILLA (EUPHORBIA CERIFERA) W AX | 0,7 |
| OCTYLDODECYL MYRISTATE | 2,0 |
| PHENOXYETHANOL, ETHYLHEXYLGLYCERIN | 0,8 |
| PARFUM | 0,3 |
| HYDROGLYCOLIC EXTRACT OF PLANT(S) | 3,0 |
| HYDROLYZED SOY PROTEIN | 2,0 |
| **TOTAL** | **100** |

### Cire coiffante

| **Nom INCI** | **% Matière** |
|---|---|
| WATER | 65,2 |
| GLYCERIN | 7,0 |
| TAPIOCA STARCH | 2,0 |
| SODIUM POLYACRYLATE | 1,0 |
| POLYGLYCERYL-6 DISTEARATE, JOJOBA ESTERS, POLYGLYCERYL-3 BEESWAX, CETYL ALCOHOL | 4,0 |
| BEESWAX | 14,0 |

| **Dérivé de cires de l'examle 3** | **5,5** |
|---|---|
| PHENOXYETHANOL, ETHYLHEXYLGLYCERIN | 0,8 |
| PARFUM | 0,5 |
| **TOTAL** | **100** |

### Soin anti-frisottis

| **Nom INCI** | **% Matière** |
|---|---|
| WATER | 71,0 |
| INULIN | 2,0 |
| SCLEROTIUM GUM | 0,5 |
| POLYGLYCERYL-6 DISTEARATE, JOJOBA ESTERS, POLYGLYCERYL-3 BEESWAX, CETYL ALCOHOL | 3,0 |
| **Dérivé de cires de l'exemple 3** | **2,0** |
| COCO-CAPRYLATE | 20,0 |
| PHENOXYETHANOL, ETHYLHEXYLGLYCERIN | 1,0 |
| PARFUM | 0,3 |
| WATER, YELLOW 5 | 0,2 |
| **TOTAL** | **100** |

### Conditionneur pour barbe

| **Nom INCI** | **% Matière** |
|---|---|
| POLYGLYCERYL-6 DISTEARATE, JOJOBA ESTERS, POLYGLYCERYL-3 BEESWAX, CETYL ALCOHOL | 3,00 |
| STEARYL HEPTANOATE, STEARYL CAPRYLATE | 7,00 |
| CAMELLIA JAPONICA SEED OIL | 10,00 |
| SIMMONDSIA CHINENSIS (JOJOBA) SEED OIL | 7,00 |
| **Dérivé de cires de l'exemple 3** | **3,50** |
| CETYL ALCOHOL | 1,50 |
| TOCOPHEROL | 0,05 |
| PHENOXYETHANOL, ETHYLHEXYLGLYCERIN | 1,00 |
| WATER | 59,05 |
| GLYCERIN | 6,00 |
| MICROCRYSTALLINE CELLULOSE, CELLULOSE GUM | 1,00 |
| XANTHAN GUM | 0,20 |
| PARFUM | 0,50 |
| PROPANEDIOL, WATER, HORDEUM VULGARE SEED EXTRACT | 0,20 |
| **TOTAL** | **100** |

## Revendications

1. Utilisation d'une composition cosmétique capillaire pour contrôler le volume capillaire comprenant un dérivé de cires obtenu par la réaction de transestérification simultanée d'un mélange comprenant de la cire de jojoba et de la cire de tournesol, en présence du polyglycérol-3.

2. Utilisation d'une composition cosmétique capillaire pour son effet anti-frisottis comprenant un dérivé de cires obtenu par la réaction de transestérification simultanée d'un mélange comprenant de la cire de jojoba et de la cire de tournesol, en présence du polyglycérol-3.

3. Utilisation d'une composition cosmétique capillaire pour le démêlage capillaire, avantageusement pour faciliter le peignage comprenant un dérivé de cires obtenu par la réaction de transestérification simultanée d'un mélange comprenant de la cire de jojoba et de la cire de tournesol, en présence du polyglycérol-3.

4. Utilisation d'une composition cosmétique capillaire pour améliorer la définition et le maintien des boucles capillaires comprenant un dérivé de cires obtenu par la réaction de transestérification simultanée d'un mélange comprenant de la cire de jojoba et de la cire de tournesol, en présence du polyglycérol-3.

5. Utilisation d'une composition cosmétique capillaire pour lutter contre l'électricité statique générée au niveau capillaire comprenant un dérivé de cires obtenu par la réaction de transestérification simultanée d'un mélange comprenant de la cire de jojoba et de la cire de tournesol, en présence du polyglycérol-3.

6. Utilisation d'une composition cosmétique capillaire pour augmenter l'hydrophobie capillaire comprenant un dérivé de cires obtenu par la réaction de transestérification simultanée d'un mélange comprenant de la cire de jojoba et de la cire de tournesol, en présence du polyglycérol-3.

7. Utilisation d'une composition cosmétique capillaire selon l'une des revendications 1 à 6, **caractérisée en ce que** la cire de tournesol est choisie parmi la cire de tournesol non hydrolysée, partiellement hydrolysée ou totalement hydrolysée.

8. Utilisation d'une composition cosmétique capillaire selon la revendication 7, **caractérisée en ce qu'**elle comprend en outre un alcool gras saturé libre ou estérifié, avantageusement contenant au moins 22 atomes de carbones et/ou un acide gras saturé, libre ou estérifié, avantageusement contenant au moins 22 atomes de carbones.

9. Utilisation d'une composition cosmétique capillaire selon l'une des revendications 7 à 8, **caractérisée** en ce le rapport massique cire de jojoba/cire de tournesol représente entre 50/50 et 90/10, avantageusement entre 60/40 et 80/20, de préférence 70/30.

10. Utilisation d'une composition cosmétique capillaire selon l'une des revendications 7 à 9, **caractérisée** en ce le rapport massique cire de jojoba et cire de tournesol/polyglycérol-3 représente entre 99/1 et 80/20, avantageusement entre 98/2 et 90/10, de préférence 95/5.

11. Utilisation d'une composition cosmétique capillaire selon l'une des revendications 7 à 10, **caractérisée** en ce le dérivé de cires représente entre 0,01 et 20% en poids de la composition, avantageusement entre 0,1 et 5%.

## Patentansprüche

1. Verwendung einer kosmetischen Zusammensetzung für die Haarpflege, um dem Haar mehr Volumen zu geben, umfassend ein Wachsderivat, erhalten durch gleichzeitige Transesterifizierung einer Mischung, die Jojoba- Wachs und Sonnenblumenwachs, in Anwesenheit von Polyglyzerin-3 enthält.

2. Verwendung einer kosmetischen Zusammensetzung für die Haarpflege aufgrund seiner Anti- Frizz- Wirkung, umfassend ein Wachsderivat, erhalten durch gleichzeitige Transesterifizierung einer Mischung, die Jojoba- Wachs und Sonnenblumenwachs, in Anwesenheit von Polyglyzerin-3 enthält.

3. Verwendung einer kosmetischen Zusammensetzung für die Haarpflege zum Entwirren der Haare, vorteilhafterweise zur Erleichterung des Kämmens, umfassend ein Wachsderivat, erhalten durch gleichzeitige Transesterifizierung einer Mischung, die Jojoba- Wachs und Sonnenblumenwachs, in Anwesenheit von Polyglyzerin-3 enthält.

4. Verwendung einer kosmetischen Zusammensetzung für die Haarpflege zur Verbesserung der Definition und Haltbarkeit von Haarlocken, umfassend ein Wachsderivat, erhalten durch gleichzeitige Transesterifizierung einer Mischung die Jojoba- Wachs und Sonnenblumenwachs, in Anwesenheit von Polyglyzerin-3 enthält.

5. Verwendung einer kosmetischen Zusammensetzung für die Haarpflege zur Einschränkung der statischen Elektrizität, die im Haar entsteht, umfassend ein Wachsderivat, erhalten durch gleichzeitige Transesterifizierung einer Mischung die Jojoba- Wachs und Sonnenblumenwachs, in Anwesenheit von Polyglyzerin-3 enthält.

6. Verwendung einer kosmetischen Zusammensetzung für die Haarpflege zur Erhöhung der Wasserabstoßung des Haares, umfassend ein Wachsderivat, erhalten durch gleichzeitige Transesterifizierung einer Mischung die Jojoba- Wachs und Sonnenblumenwachs, in Anwesenheit von Polyglyzerin-3 enthält.

7. Verwendung einer kosmetischen Zusammensetzung für die Haarpflege nach einem der Ansprüche 1 bis 6, **dadurch gekennzeichnet, dass** das Sonnenblumenwachs ausgewählt wird aus nicht hydrolysiertem, teilweise hydrolysiertem oder vollständig hydrolysiertem Sonnenblumenwachs.

8. Verwendung einer kosmetischen Zusammensetzung für die Haarpflege nach Anspruch 7, **dadurch gekennzeichnet, dass** sie außerdem einen freien oder veresterten gesättigten Fettalkohol enthält, der vorteilhafterweise mindestens 22 Kohlenstoffatome und/ oder eine freie oder veresterte gesättigte Fettsäure enthält, die vorteilhafterweise mindestens 22 Kohlenstoffatome enthält.

9. Verwendung einer kosmetischen Zusammensetzung für die Haarpflege nach einem der Ansprüche 7 bis 8, **dadurch gekennzeichnet, dass** das Masseverhältnis Jojoba- Wachs/ Sonnenblumenwachs zwischen 50/50 und 90/10 beträgt, vorteilhafterweise zwischen 60/40 und 80/20, am besten 70/30.

10. Verwendung einer kosmetischen Zusammensetzung für die Haarpflege nach einem der Ansprüche 7 bis 9, **dadurch gekennzeichnet, dass** das Masseverhältnis Jojoba- Wachs/ Sonnenblumenwachs zwischen 99/1 und 80/20 beträgt, vorteilhafterweise zwischen 98/2 und 90/10, am besten 95/5.

11. Verwendung einer kosmetischen Zusammensetzung für die Haarpflege nach einem der Ansprüche 7 bis 10, **dadurch gekennzeichnet, dass** das Wachsderivat zwischen 0,01 und 20 Gewichts-% der Zusammensetzung darstellt, am besten zwischen 0,1 und 5%.

## Claims

1. Use of a cosmetic hair care composition for controlling hair volume comprising a derivative of waxes obtained by the reaction of simultaneous transesterification of a mixture comprising jojoba wax and sunflower wax, in the presence of polyglycerol-3.

2. Use of a cosmetic hair care composition for its anti-frizz effect comprising a derivative of waxes obtained by the reaction of simultaneous transesterification of a mixture comprising jojoba wax and sunflower wax, in the presence of polyglycerol-3.

3. Use of a cosmetic hair care composition for untangling hair, advantageously in order to facilitate combing, comprising a derivative of waxes obtained by the reaction of simultaneous transesterification of a mixture comprising jojoba wax and sunflower wax, in the presence of polyglycerol-3.

4. Use of a cosmetic hair care composition for improving the definition and hold of curls in the hair, comprising a derivative of waxes obtained by the reaction of simultaneous transesterification of a mixture comprising jojoba wax and sunflower wax, in the presence of polyglycerol-3.

5. Use of a cosmetic hair care composition for counteracting static electricity generated in the hair, comprising a derivative of waxes obtained by the reaction of simultaneous transesterification of a mixture comprising jojoba wax and sunflower wax, in the presence of polyglycerol-3.

6. Use of a cosmetic hair care composition for increasing the hydrophobic nature of the hair, comprising a derivative of waxes obtained by the reaction of simultaneous transesterification of a mixture comprising jojoba wax and sunflower wax, in the presence of polyglycerol-3.

7. Use of the cosmetic hair care composition as claimed in one of claims 1 to 6, **characterized in that** the sunflower wax is selected from non-hydrolysed, partially hydrolysed or completely hydrolysed sunflower wax.

8. Use of the cosmetic hair care composition as claimed in claim 7, **characterized in that** it furthermore comprises a free or esterified saturated fatty alcohol advantageously containing at least 22 carbon atoms and/or a free or esterified saturated fatty acid advantageously containing at least 22 carbon atoms.

9. Use of the cosmetic hair care composition as claimed in one of claims 7 to 8, **characterized in that** the weight ratio of jojoba wax/sunflower wax represents between 50/50 and 90/10, advantageously between 60/40 and 80/20, preferably 70/30.

10. Use of the cosmetic hair care composition as claimed in one of claims 7 to 9, **characterized in that** the weight ratio of j oj oba wax and sunflower wax/polyglycerol-3 represents between 99/1 and 80/20, advantageously between 98/2 and 90/10, preferably 95/5.

11. Use of the cosmetic hair care composition as claimed in one of claims 7 to 10, **characterized in that** the derivative of waxes represents between 0.01% and 20% by weight of the composition, advantageously between 0.1% and 5%.
